# EUROPEAN PATENT SPECIFICATION

(11) **EP 2 612 152 B1**
(45) Date of publication and mention of the grant of the patent: **10.06.2020**
(21) Application number: 11748961.7
(22) Date of filing: 30.08.2011
(51) Int. Cl.: G01N 33/68

(54) **METHOD TO DIAGNOSE INFECTIOUS PERITONITIS AND PREDICT THE SEVERITY AND OUTCOME THEREOF IN HUMANS.**
VERFAHREN ZUR DIAGNOSE ANSTECKENDER PERITONITIS UND VORHERSAGE DER SCHWEREGRAD UND AUSGANG DAVON IN MENSCHEN.
MÉTHODE POUR DIAGNOSTIQUER LA PERITONITE INFECTIEUSE ET POUR PRÉDIRE LA GRAVITÉ ET L'ISSUE DE CELLE-CI CHEZ L'HOMME

(30) Priority: 31.08.2010 EP 10174641
(43) Date of publication of application: 10.07.2013
(73) Proprietor: Otto-von-Guericke-Universität Magdeburg, 39120 Magdeburg (DE); Universität Zürich, 8006 Zürich (CH)
(72) Inventor: HALANGK, Walter, 39167 Niederndodeleben (DE); GRAF, Rolf, CH-8051 Zürich (CH); GUKASJAN, Raphael, 39116 Magdeburg (DE); SCHULZ, Hans-Ulrich, 39128 Magdeburg (DE)
(74) Representative: Latscha Schöllhorn Partner AG
(86) International application number: PCT/EP2011/064854
(87) International publication number: WO 2012/028594

(56) References cited:
- WO-A1-2009/030456
- SU-A1- 1 560 084

### SEQUENCE LISTING

<110> Otto-von-Guericke-Universitaet Magdeburg
   Universitaet Zuerich
   Halangk, Walter
   Graf, Rolf
<120> Method for assaying peritonitis in humans
<130> P389A
<150> EP10174641.0
   <151> 2010-08-31
<160> 8
<170> PatentIn version 3.5
<210> 1
   <211> 166
   <212> PRT
   <213> Homo sapiens
<400> 1
   Met Ala Gln Thr Ser Ser Tyr Phe Met Leu Ile Ser Cys Leu Met Phe
   1               5                   10                  15
   Leu Ser Gln Ser Gln Gly Gln Glu Ala Gln Thr Glu Leu Pro Gln Ala
               20                  25                  30
   Arg Ile Ser Cys Pro Glu Gly Thr Asn Ala Tyr Arg Ser Tyr Cys Tyr
           35                  40                  45
   Tyr Phe Asn Glu Asp Arg Glu Thr Trp Val Asp Ala Asp Leu Tyr Cys
       50                  55                  60
   Gln Asn Met Asn Ser Gly Asn Leu Val Ser Val Leu Thr Gln Ala Glu
   65                  70                  75                  80
   Gly Ala Phe Val Ala Ser Leu Ile Lys Glu Ser Gly Thr Asp Asp Phe
                   85                  90                  95
   Asn Val Trp Ile Gly Leu His Asp Pro Lys Lys Asn Arg Arg Trp His
               100                 105                 110
   Trp Ser Ser Gly Ser Leu Val Ser Tyr Lys Ser Trp Gly Ile Gly Ala
           115                 120                 125
   Pro Ser Ser Val Asn Pro Gly Tyr Cys Val Ser Leu Thr Ser Ser Thr
       130                 135                 140
   Gly Phe Gln Lys Trp Lys Asp Val Pro Cys Glu Asp Lys Phe Ser Phe
   145                 150                 155                 160
   Val Cys Lys Phe Lys Asn
                   165
<210> 2
   <211> 166
   <212> **PRT**
   <213> Homo sapiens
<400> 2
   Met Ala Gln Thr Asn Ser Phe Phe Met Leu Ile Ser Ser Leu Met Phe
   1               5                   10                  15
   Leu Ser Leu Ser Gln Gly Gln Glu Ser Gln Thr Glu Leu Pro Asn Pro
               20                  25                  30
   Arg Ile Ser Cys Pro Glu Gly Thr Asn Ala Tyr Arg Ser Tyr Cys Tyr
           35                  40                  45
   Tyr Phe Asn Glu Asp Pro Glu Thr Trp Val Asp Ala Asp Leu Tyr Cys
       50                  55                  60
   Gln Asn Met Asn Ser Gly Asn Leu Val Ser Val Leu Thr Gln Ala Glu
   65                  70                  75                  80
   Gly Ala Phe Val Ala Ser Leu Ile Lys Glu Ser Ser Thr Asp Asp Ser
                   85                  90                  95
   Asn Val Trp Ile Gly Leu His Asp Pro Lys Lys Asn Arg Arg Trp His
               100                 105                 110
   Trp Ser Ser Gly Ser Leu Val Ser Tyr Lys Ser Trp Asp Thr Gly Ser
           115                 120                 125
   Pro Ser Ser Ala Asn Ala Gly Tyr Cys Ala Ser Leu Thr Ser Cys Ser
       130                 135                 140
   Gly Phe Lys Lys Trp Lys Asp Glu Ser Cys Glu Lys Lys Phe Ser Phe
   145                 150                 155                 160
   Val Cys Lys Phe Lys Asn
                   165
<210> 3
   <211> 27
   <212> DNA
   <213> Artificial sequence
<220>
   <223> Synthetic construct, forward primer for PSP/reg 1 alpha
<400> 3
   gaaaagacaa gaggcccaga cagagtt 27
<210> 4
   <211> 31
   <212> DNA
   <213> Artificial sequence
<220>
   <223> Synthetic construct, elongation primer for PSP/reg 1 alpha
<400> 4
   gtatctctcg agaaaagaca agaggcccag a 31
<210> 5
   <211> 21
   <212> DNA
   <213> Artificial sequence
<220>
   <223> Synthetic construct, reverse primer for PSP/reg 1 alpha
<400> 5
   ctagtttttg aacttgcata c 21
<210> 6
   <211> 28
   <212> DNA
   <213> Artificial sequence
<220>
   <223> Synthetic construct, forward primer for PSP/reg 1 beta
<400> 6
   gaaaagacag gagtcccaga cagagctg 28
<210> 7
   <211> 32
   <212> DNA
   <213> Artificial sequence
<220>
   <223> Synthetic construct, elongation primer for PSP/reg 1 beta
<400> 7
   gtatctctcg agaaaagaca ggagtcccag ac 32
<210> 8
   <211> 37
   <212> DNA
   <213> Artificial sequence
<220>
   <223> Synthetic construct, reverse primer for PSP/reg 1 beta
<400> 8
   atctgcagtc tagaattctg caggaccagt tctagac 37

## Description

### Field of the Invention

The present invention relates to a method of prediction and/or diagnosis of an intra-abdominal infection in human, in particular for prediction of the development of infectious peritonitis and intra-abdominal abscess, based on the level of pancreatic stone protein / regenerating protein (PSP/reg) in body fluids. The invention allows sorting patients according to risk.

### Background of the invention

Peritonitis denotes inflammation of the peritoneum from any cause, infectious or not. It may be regarded as a localized equivalent of the systemic inflammatory response syndrome (SIRS). Peritonitis is defined according to both its cause and extent. Intra-abdominal infections denote peritonitis caused by bacteria (e.g., a local inflammatory process initiated by bacteria and their toxins) and may be regarded as the localized equivalent of systemic sepsis. They are characterized by the broad variety in causes and severity of the infection. Intra-abdominal infectious complications are an important cause of patient morbidity and mortality in the intensive care unit (ICU).

Infectious peritonitis can be classified as primary, secondary, or tertiary. In primary peritonitis (also called spontaneous bacterial peritonitis), the source of infection does not arise from the gastrointestinal tract, and there is no identifiable anatomical derangement of the intra-abdominal viscera. Primary peritonitis is mostly caused by a chronic liver disease, such as cirrhosis. In contrast, secondary peritonitis is due to an infection of the abdominal viscera, and may arise as a consequence of perforation, ischemic necrosis, or penetrating injury. Tertiary peritonitis is defined as peritonitis that persists or recurs after more than one failed source control procedure, and is highly frequent in patients requiring intensive care unit admission for severe abdominal infections.

It is important for physicians to discriminate patients suffering from a local intra-abdominal infection from patients suffering from an intra-abdominal infection with a systemic reaction (abdominal sepsis). Up to 40% of patients with peritonitis may develop abdominal sepsis. Because of the heterogeneous pattern of abdominal sepsis, it is difficult to precisely define the disease and appraise its severity. Hence accurate diagnosis is markedly hampered.

From a clinical viewpoint, one distinguishes two major types of intra-abdominal infections: uncomplicated and complicated. In uncomplicated intra-abdominal infection, the infectious process involves a single organ and no anatomical disruption occurs. In contrast, in complicated intra-abdominal infections, the infectious process progresses beyond the organ that is the source of infection, and causes either localised peritonitis, also referred to as abdominal abscess (i.e. an intra-abdominal infection that has been confined within the abdominal cavity), or diffuse peritonitis (i.e. uncontained spread of infection). Diffuse peritonitis is characterized by high mortality and necessitates urgent celiotomy.

Currently, diagnosis of peritonitis is mainly clinical, in particular for surgical cases (e.g. appendicitis, perforated peptic ulcer, anastomotic leak post-surgery, strangulated bowel). Diagnosis of cases with ascites requires paracentesis for polymorphonuclear (PMN) cell count, which is currently the gold standard method for spontaneous bacterial peritonitis. Other detection methods, such as leukocyte esterase reagent strips or lactoferrin, are being introduced. Unfortunately, the need to sample ascites is a major drawback for most non-clinical diagnostic techniques. Indeed, this method (i) requires presence of ascites, (ii) demands time-consuming analysis, (iii) involves repetitive and extra invasive procedures (paracentesis). Moreover, the current method used to diagnose spontaneous bacterial peritonitis is based on a manual count of ascitic fluid PMNs, a time-consuming and costly procedure that is not always timely available on emergency settings. Therefore, there is a need for new diagnostic methods based on serum, i.e. that would not require paracentesis.

Pancreatic stone protein/regenerating protein (PSP/reg) belongs to a family of lectin-binding proteins that were identified initially in patients with pancreatitis (L. Multigner et al., Gastroenterology 1985, 89:387-391). PSP/reg has been studied predominantly in the pancreas. Under conditions of acute or chronic pancreatitis, it is highly up-regulated and may appear in the serum (W. Schmiegel et al., Gastroenterology 1990, 99:1421-1430). Serum levels are also raised in several gastrointestinal diseases (Satomura et al., J. Gastroenterol 1995, 30:643-650). The function of PSP/reg is still highly debated, but it is generally assumed that it is involved in promoting cell proliferation during regenerative processes (Y. Kinoshita et al., J. Gastroenterol 2004, 39:507-513).

Although this protein is a secretion product, its expression is not induced by diet alone. In trauma patients, it has been shown that PSP/reg is up-regulated in blood after trauma, and that the PSP/reg level is related to the severity of inflammation. In particular, it is highly increased in patients during sepsis (M. Keel et al., Crit Care Med. 2009, 37(5):1642-1648).

The PSP/reg level in blood serum was recently proven to be a reliable indicator of systemic sepsis (WO 2009/030456). Patent application SU 1 560 084 discloses methods to diagnose peritonitis involving the measurement of the systolic and venous blood pressure in the small intestines.

### Summary of the invention

The present disclosure relates to a method of prediction and/or diagnosis of an intra-abdominal infection in humans, in particular for prediction of the development of peritonitis caused by bacteria or virus, and related clinical complications and outcome such as subsequent organ failure and death, wherein the level of pancreatic stone protein / regenerating protein (PSP/reg) is determined in body fluid sample, and a high level is indicative of the development and the severity of infectious peritonitis sepsis at early stages of the disease. In addition, in the present disclosure, high levels of PSP/reg are predictive of survival in peritonitis patients and correlate with clinical severity scores.

The present invention is defined according to the appended claims.

### Brief description of the Figures

*Figure 1**: Determination of PSP*/*reg values in sera of patients at admission to the hospital with a suspicion of peritonitis.*
   C = control, P = peritonitis, N = none, L = localized, D = diffuse, S = survivors, NS = non-survivors.
   Patients were retrospectively categorized:
   Figure 1a: patients with no infectious peritonitis (C, n=44), and patients with infectious peritonitis (P, n=88). PSP/reg values (log₁₀ ng/mL) of the three groups are plotted as box plots with the mean and the 95% confidence interval. Statistical analysis was performed using parametric method (unpaired t test); p = significance.
   Figure 1b: patients with no infection (N, n=44), patients with local intra-abdominal infection (L, n=24) and patients with a diffuse intra-abdominal infection (D, n=64). PSP/reg values (log₁₀ ng/mL) of the three groups are plotted as box plots with the mean and the 95% confidence interval. Statistical analysis was performed using parametric method (unpaired t test); p = significance, *p = p values for diffuse infection vs. no infection.
   Figure 1c: patients with no organ failure (0, n=69), patients with failure of 1 to 3 organs (1-3, n=57) and patients with failure of more than 3 organs (>3, n=6). PSP/reg values (log₁₀ ng/mL) of the three groups are plotted as box plots with the mean and the 95% confidence interval. Statistical analysis was performed using parametric method (unpaired t test); p = significance, *p = p values for >3 organ failure vs. no organ failure.
   Figure 1d: survivors (S, n=107), non-survivors (NS, n=25). PSP/reg values (log₁₀ ng/mL) of the two groups are plotted as box plots with the mean and the 95% confidence interval. Statistical analysis was performed using parametric method (unpaired t test); p = significance.
*Figure 2**: Receiver operating characteristic curves*
   x-axis: 1-specificity, y-axis: sensitivity
   Figure 2a: Receiver operating characteristic curve for the capacity of PSP to discriminate between patients without (n= 44) and with (n= 88) infectious peritonitis.
   Figure 2b: Receiver operating characteristic curve for the capacity of PSP to discriminate between patients without (n= 69) and with (n= 63) organ failure
   Figure 2c: Receiver operating characteristic curve for the capacity of PSP to discriminate between survivors (n= 107) and non-survivors (n= 25) of infectious peritonitis.
*Figure 3**: Correlation of APACHE score and PSP*/*reg serum levels at admission to the ICU (p<0.001) in 137 patients.*
   PSP/reg was logarithmized for graphical demonstration. x-axis: APACHE score, y-axis: PSP/reg (log₁₀ ng/ml).

### Detailed description of the invention

The present invention relates to a method of prediction and/or diagnosis of an intra-abdominal infection in humans, in particular for prediction of the development of peritonitis of infectious origin, wherein the level of pancreatic stone protein / regenerating protein (PSP/reg) is determined in a body fluid sample, e.g. serum or plasma, and a high level is indicative of the development and the severity of infectious peritonitis at early stages of the disease. In particular, a high level is indicative of the development of a infectious peritonitis while an intermediate level is indicative of the development of an intra-abdominal abscess.

As defined herewith, "peritonitis" is an inflammation of the peritoneal membrane caused by either infectious or non-infectious reasons. The vast majority of clinically significant peritonitis is of infectious origin caused by bacteria.

As defined herewith, "intra-abdominal infection" denotes peritonitis caused by bacteria (e.g., a local inflammatory process initiated by bacteria and their toxins) or virus. Infectious peritonitis may be regarded as the localized equivalent of systemic sepsis. Because of the heterogeneous pattern of abdominal sepsis (the term generally given to sepsis that originates from the abdomen), it is difficult to define the disease precisely or appraise its severity, thereby impeding the diagnostic progress. It is important for physicians to discriminate patients suffering from a local intra-abdominal infection from those suffering from an intra-abdominal infection with a systemic reaction.

Infectious peritonitis can be classified as primary, secondary, or tertiary. In primary peritonitis (also called spontaneous bacterial peritonitis), the source of infection does not arise from the gastrointestinal tract, and there is no identifiable anatomical derangement of the intra-abdominal viscera. Primary peritonitis is mostly caused by a chronic liver disease, such as cirrhosis. In contrast, secondary peritonitis is due to an infection of the abdominal viscera, and may arise as a consequence of perforation, ischemic necrosis, or penetrating injury. Tertiary peritonitis is defined as peritonitis that persists or recurs after more than one failed source control procedure, and is highly frequent in patients requiring intensive care unit admission for severe abdominal infections.

One should still distinguish sepsis from peritonitis, since, in sepsis, the origin of infection is not ubiquitous and its presence is wide, while peritonitis implies a local, intra-abdominal, inflammation / infection.

In a first aspect, the invention relates to an *ex vivo* method for detecting and/or diagnosing an intra-abdominal infection in humans. In particular, the present method according to the invention allows the detection of the development and the prediction of the severity of peritonitis of infectious origin. It also allows the prognosis of peritonitis of infectious origin. The methods according to the present invention comprise determining the level of PSP/reg in an isolated body fluid sample, e.g. serum or plasma, wherein said level is indicative of the severity of infectious peritonitis at early stages of the disease and of the complications and risk of mortality linked to said infections.

As defined herewith, "patient" refers to any mammalian animal including human, dog, cat, cattle, goat, pig, swine, sheep and monkey. Patients are preferably humans.

As defined herewith, "PSP/reg" refers to human pancreatic stone protein, also called regenerating gene (REG) I protein or lithostatine or pancreatic thread protein (Gross et al., J. Clin. Invest. 1985, 76:2115-2126) and can be the isoform alpha (Uniprot sequence number: P05451, also identified herewith as SEQ ID NO:1) or beta (Uniprot sequence number: P48304, also identified herewith as SEQ ID NO:2).

As defined herewith, "body sample" refers to any sample that is obtained from the patient's body. Body sample includes body fluid samples and extracts from solid tissue or from fecal matter. Body fluid samples include, for instance, samples of whole blood, serum, plasma, urine, sputum, cerebrospinal fluid, tear fluid, sweat, or milk.

One aspect of the invention relates to a method of prognosis and/or diagnosis of peritonitis in a patient, preferably a human, wherein the level of pancreatic stone protein / regenerating protein (PSP/reg) is determined in a body fluid sample or in a solid body tissue, and a high level is indicative of the development and the severity of the disease.

In a further aspect of the method of the invention, the peritonitis is caused by bacteria, viruses, fungi, or parasites, and is primary, secondary, or tertiary peritonitis.

In another aspect, the present invention is directed to an *ex vivo* method of prognosis and/or diagnosis of intra-abdominal infections in a patient, comprising determining the level of pancreatic stone protein / regenerating protein (PSP/reg) in a body fluid sample from said patient. The patient is preferably a human.

In a preferred aspect, the method of the invention is for detecting the development of peritonitis in a patient.

In another aspect of the method of the invention, the PSP/reg protein has the amino acid sequence of SEQ ID NO:1 or SEQ ID NO:2.

According to a further aspect, the method of the invention is an *in vitro* diagnostic method.

In a still further aspect, the present invention relates to an ex vivo method of prognosis and/or diagnosis of intra-abdominal infections in a patient, or to an *exvivo* method for detecting the development of peritonitis in a patient, comprising:
a) providing a body fluid sample from said patient;
b) determining the level of PSP/reg in said sample;
c) comparing the level of PSP/reg determined in step b) with a reference value;
wherein a higher level of PSP/reg determined in step b), compared to the reference value, is indicative of the severity of the intra-abdominal infection and/or peritonitis and predictive of the outcome.

In a specific aspect of the invention, the reference value is the level of PSP/reg measured in a body fluid sample from a patient without known or suspected infection.

In another specific aspect of the invention, the body fluid sample is serum or plasma.

In another aspect of the invention, the reference value is about 20 ng/ml PSP/reg in serum or plasma.

PSP/reg levels, indicative of development and severity of peritonitis, are dependent on the body fluid chosen for determination. Serum or plasma levels are between 25 and 30 ng/ml at admission to the intensive care units (ICU) in patients with suspicion of peritonitis.

More precisely, the invention relates to a method of prediction and/or diagnosis of the development of peritonitis, wherein the level of PSP/reg is determined in serum or plasma, and a level of 25 ng/ml or more, in particular a level of 30 ng/ml or more, at admission to the ICU with suspicion of peritonitis or following abdominal surgery, is indicative of the development of a local or diffuse peritonitis, wherein a level of 50 ng/ml or more is indicative of organ failure, and wherein a level of 400 ng/ml or more indicates that the patient is at high risk of death.

Other body fluids than serum or plasma useful for determination of PSP/reg levels are e.g. whole blood, urine, sputum, cerebrospinal fluid, tear fluid, sweat, milk, or extracts from solid tissue or from fecal matter.

In a preferred aspect of the *ex vivo* method of the invention, the level of PSP/reg determined in step b) is determined on the day of admission to the ICU and on subsequent days.

In an alternative preferred aspect of the *ex vivo* method of the invention, the level of PSP/reg determined in step b) is determined within the 24 hours following abdominal surgery.

In a further aspect of the method of the invention, the level of PSP/reg determined in step b) is determined within the 24 hours following abdominal surgery and allows the triage of the patients according to risk, wherein a level below 30 ng/ml is indicative that the patient can be placed in an intermediate, or normal care unit after surgery, and wherein a level of 100 ng/ml or more indicates that the patient requires intensive care and must undergo an infection workup or new surgery to clean up the infection site.

Any known method may be used for the determination of the level of PSP/reg in body fluids. Methods considered are e.g. Enzyme-linked immunosorbent assay (ELISA), Radioimmunoassay (RIA), Enzymoimmunoassay (EIA), mass spectrometry, or microarray analysis. Such methods when used for the detection of the development of local or systemic infection, in particular of the detection of the development of sepsis, are a further object of the invention.

A preferred method for the determination of PSP/reg in human body fluids, e.g. serum or plasma, is an ELISA. In such an embodiment, the PSP/reg ELISA consists of a sandwich array: Conventional microtiter plates are coated with one type of antibody ("first" antibody), directed against PSP/reg. The plates are then blocked and the sample or standard is loaded. After the incubation, a different type of antibody ("second" antibody) against PSP/reg is applied. A third antibody detecting the particular type of the "second" antibody, conjugated with a suitable label, e.g. an enzyme for chromogenic detection, is then added. Finally the plate is developed with a substrate for the label in order to detect and quantify the label, being a measure for the presence and amount of PSP/reg. If the label is an enzyme for chromogenic detection, the substrate is a colour-generating substrate of the conjugated enzyme. The colour reaction is then detected in a microplate reader and compared to standards.

Suitable pairs of antibodies ("first" and "second" antibody) are any combination of guinea pig, rat, mouse, rabbit, goat, chicken, donkey, or horse antibodies. Preferred are polyclonal antibodies, but it is also possible to use monoclonal antibodies or antibody fragments. Most preferred are monoclonal antibodies. Suitable labels are chromogenic labels, i.e. enzymes which can be used to convert a substrate to a detectable coloured or fluorescent compound, spectroscopic labels, e.g. fluorescent labels or labels presenting a visible colour, affinity labels which may be developed by a further compound specific for the label and allowing easy detection and quantification, or any other label used in standard ELISA.

Other preferred methods of PSP/reg detection are radioimmunoassay or competitive immunoassay using a single antibody and chemiluminescence detection on automated commercial analytical robots. Microparticle enhanced fluorescence, fluorescence polarized methodologies, or mass spectrometry may also be used. Detection devices, e.g. microarrays, are useful components as readout systems for PSP/reg.

PSP/reg is a protein expressed in the pancreas and the intestine. It can be cloned from pancreatic mRNA and subcloned into a yeast expression vector. The protein can then be expressed under the control of Alcohol Dehydrogenase promoter (ADH). A suitable expression medium may comprise methanol to induce and maintain the secretion of PSP/reg. PSP/reg is preferably purified using SP-Sepharose-cellulose by a pH and salt gradient. Such purified PSP/reg is used to prepare standard solutions for comparison with PSP/reg levels in body fluids. Polyclonal antibodies against the protein may be obtained from mice, rats, rabbits, goats, chicken, donkey, horses and guinea pigs or other suitable animals using standard methods.

The invention further relates to a kit of parts for the determination of PSP/reg for diagnosis/prediction of intra-abdominal infections comprising, for example, apparatus, reagents and standard solutions of PSP/reg. Apparatus considered are e.g. microtiter plates for ELISA, pre-coated ELISA plates, and plate covers. Reagents are those reagents particularly developed and designed for the detection of PSP/reg. Standard solutions of PSP/reg preferably contain PSP/reg synthesized according to the directions hereinbelow. The kit of parts may contain further hardware, such as pipettes, solutions such as buffers, blocking solutions and the like, filters, colour tables and directions for use.

In another aspect, the invention relates to a kit for detecting intra-abdominal infection in patients according to a method of the invention comprising at least one antibody directed against PSP/reg and reagents.

In another aspect, the invention relates to a kit for predicting the severity of a peritonitis infection in a patient and patient outcome according to a method of the invention comprising at least one antibody directed against PSP/reg and reagents.

In a further aspect, the invention relates to the use of a kit according to the invention for detecting the development of a local or diffuse abdominal infection in a patient, notably the development of peritonitis, more notably the development of sepsis, for example, in a method according to the invention.

Antibodies directed against PSP/reg can be produced by standard methods in the field including polyclonal antibody production and monoclonal antibody production. Preferably, the antibodies are directed against a PSP/reg protein having the amino acid sequence of SEQ ID NO:1 or SEQ ID NO:2. More preferably, the antibodies are mouse IgG1 monoclonal antibodies.

The reason for the increase in PSP/reg serum levels during early development of infectious peritonitis is not fully understood. In rats, an increase in PSP/reg synthesis in the absence of pancreatic damage is observed after stress (e.g., anesthesia). In a recent study, an increase in PSP/reg in correlation with the development of severe sepsis was observed in a set of human patients with severe trauma but apparent absence of pancreatic damage (M. Keel et al., Crit Care Med. 2009, 37(5):1642-1648). The data were analyzed using stratification assigned to trauma patients without infection, patients with infection and patients with sepsis. PSP/reg values in patients without an infection were slightly increased. In patients with polytrauma and infection, PSP/reg levels were further increased. Finally, polytraumatic patients exhibiting sepsis showed a large increase in serum PSP/reg. The appearance of PSP/reg in blood serum would imply an altered pathway, diverting the protein from pancreatic juice into the blood. It has also been shown that members of the lectin binding family (e.g. pancreatitis-associated protein) are inducible by cytokines. There is a strong and concerted action of cytokines after trauma. The complexity of the cytokine response, with many different cytokines being released, is not understood. Thus it is likely that PSP/reg reacts to cytokines that are raised under condition of systemic stress or trauma. In contrast, other pancreatic enzymes, e.g. amylase and lipase, appear not to be regulated by cytokines, their appearance in the blood being a result of diversion only. The PSP/reg level in blood serum was recently proven to be a reliable indicator of systemic sepsis. Now PSP/reg levels in blood have also proven to be a reliable indicator of infectious peritonitis. The increase of PSP/reg in blood might imply a specific stress response.

It has recently been shown that unlike other indicators of inflammation, levels of PSP/reg are highly increased in patients while or before clinical signs of systemic sepsis are apparent. It is presently shown that patients developing peritonitis, a local inflammation of the peritoneal membrane, have elevated levels of PSP/reg. Particularly, PSP/reg is highly increased in patients that develop peritonitis with an increased risk of mortality.

The detection and quantification of serum PSP/reg is accomplished e.g. by a sandwich ELISA with a limit of detection of less than 100 pg/ml. Normal serum values are between 5 and 20 ng/ml. Serum values correlate with the severity of peritonitis. They may reach over 200 ng/ml before clinical signs of abdominal sepsis are available. These values allow to predict whether a patient will develop peritonitis, whether it will lead to organ failure, and to which extent it will be severe and life threatening, and hence the need for intensive treatment including costly antibiotic treatment and a stay in the intensive care unit (ICU). Compared to commercially available diagnostic assays, the PSP/reg ELISA is a reliable assay to predict severity and outcome in peritonitis patient, particularly to predict if such patient has a good or a poor chance of survival.

The methods of the invention and/or the kit according to the invention are useful for sorting the patients according to groups of risks regarding severity and outcome of the infection disease.

In peritonitis patients, a PSP/reg level, preferably from a sample obtained from the serum or plasma, below or equal to 30 ng/ml is predictive of a good chance of survival, whereas a PSP/reg level higher than or equal to 200 ng/ml, preferably higher than 250 ng/ml, more preferably higher than or equal to 300 ng/ml, more preferably higher than or equal to 400 ng/ml is associated with a high risk of mortality that could occur within a variable period comprised between 1 day to 3 years, for instance within 28 days after hospitalization.

As a consequence, regular and intensive follow-up and care, possibly including antibiotic treatment and prolonged stay, should be applied to peritonitis patients belonging to the group(s) of high risk(s).

In an abdominal surgery patients, a PSP/reg level, preferably from a sample obtained from the serum or plasma within 24 hours following the surgery procedure, below or equal to 30 ng/ml is predictive of a good chance of survival, whereas a PSP/reg level higher than or equal to 50 ng/ml, preferably higher than 80 ng/ml, more preferably higher than or equal to 100 ng/ml, more preferably higher than or equal to 200 ng/ml is associated with a high risk of infection-related post surgery complications that could occur within a variable period comprised between 1 day to 10 days after the surgery. As a consequence, decision based on PSP/reg levels as to apply regular, intermediate, or intensive follow-up and care, possibly including antibiotic treatment, prolonged stay or surgery, should be made to peritonitis patients belonging to the group(s) of high risk(s).

### Examples

### Isolation and subcloning of PSP/reg

In order to obtain cDNA for the production of PSP/reg specific antibodies, such cDNA is prepared by reverse transcription of pancreatic mRNA using state of the art laboratory methods. A PCR reaction using primers specific for the sequence coding for PSP/reg and selectively amplifying PSP/reg cDNA is performed. The PCR reaction is then repeated with the elongation primer to add a sequence specific for insertion into the *Pichia pastoris* transfection vector. The primer is designed to fuse the coding region of the signal peptide of the alpha-mating factor with a KEX2 site and the coding region of the mature human PSP/reg. Subcloning into the *Pichia pastoris* vector is a two-step procedure. First the PCR product is ligated into the pCR2.1 vector (Invitrogen, TAcloning) and the sequence verified. Then the PCR product is cleaved by Xhol/Notl restriction digestion and ligated into transfer vector pPIC9 (Invitrogen). The *Pichia pastoris* strain KM71 (Invitrogen) is transformed and the most productive clone is selected for expansion and production of recombinant protein.

### Primers used for PCR amplification and subcloning

Human PSP/reg/reg1 alpha
   Forward primers
   Reverse
Human PSP/reg/reg1 beta
   Forward primers
   Reverse primer

### Large scale expression of protein

Using a single colony, 25 ml of BMG (buffered minimal glycerol, 100 mM potassium phosphate pH 6.0, 1.34% yeast nitrogen base, 4x10⁻⁵% biotin, 1% glycerol) is inoculated in a 250 ml baffled flask and grown at 29°C in a shaking incubator (300 rpm) overnight. 10 ml of this culture is used to inoculate 1 liter of BMG in a 3 liter baffled flask and grown at 29°C (300 rpm) overnight. The cells are harvested by centrifugation at 1500-3000xg for 5 minutes at room temperature. Expression is induced by resuspending the cells in 1/5 volume (200 ml) of BMM (buffered minimum methanol, BMG in which glycerol is replaced by 0.5% methanol) in the same baffled flask. 100% methanol is added to achieve a concentration of 0.5% (1 ml) every 24 hrs until optimal time of induction is reached. The cells are harvested by centrifugation at 1500-3000xg at room temperature. The medium supernatant is collected and frozen until purification of the peptide.

The polypeptide is purified from media supernatants. Media supernatants are diluted 1:3 with distilled water. The pH is adjusted to pH 3.5 with HCI. The medium supernatant is then applied to a SP-Sepharose column and eluted by a salt and pH gradient (10 mM LiCI, 50 mM MES, pH 5.3 starting buffer, 2 M LiCI, 50 mM MES, pH 6.3 end buffer). Fractions are collected and analyzed by SDS-gel electrophoresis. The fractions with the highest and purest protein contents are combined and dialyzed against 10 mM HEPES pH 7.5. The sequence of the polypeptide is verified by N-terminal sequencing and the concentration is assessed by amino acid analysis.

### PSP/reg ELISA

In order to determine total PSP/reg, a sandwich ELISA may be used on the basis of a guinea pig antiserum raised against recombinant human PSP/reg and a rabbit antiserum against the same protein. To improve the specificity and sensitivity of the rabbit antibody, IgGs are purified by absorption on a column of protein A beads (HiTrap®, Pharmacia): A HiTrap® column is equilibrated with 200 mM NaH₂PO₄/Na₂HPO₄ at pH 7. The rabbit antiserum is pH-adjusted with the same buffer solution (final concentration 20 mM) and then loaded onto the column, which is afterwards washed with 100 mM and 10 mM Tris/HCI pH 8 consecutively. The IgG fraction is eluted with 0.1 M citric acid pH 3. The eluted fractions are immediately neutralized with 1 M Tris/HCI pH 8.9.

96-well microtiter plates (Costar EIA plates, flat bottom, high binding) are coated over night at 4°C with guinea pig anti-rat PSP/reg IgG fraction, diluted 1:500 in TBS (100 µl/well). After a washing step, the plate is blocked with 150 µl 1% BSA/TBS for one hour, which is afterwards replaced by 100 µl of different standard concentrations of recombinant human PSP/reg (0, 0.1, 0.5, 1.0, 1.5, 2.5, 3.5, or 5.0 ng/ml) or 100 µl samples of diluted sample. Samples and standards are loaded in duplicates and incubated for 1 hr at room temperature. After repeated washing, the plate is incubated for 1 hr with 100 µl rabbit anti-rat PSP/reg IgG, diluted 1:500. Another washing step follows before a 30 min incubation with 100 µl of a commercially available mouse monoclonal anti-rabbit IgG antibody is started (mouse anti-rabbit alkaline phosphatase conjugated, IgG fraction, diluted 1:1000; purchased from Sigma). The plate is then washed again, and a soluble phosphatase substrate, p-nitrophenyl phosphate disodium (Sigma 104® tablets), added in alkaline phosphatase buffer (100 mM Tris/HCI pH 9.5, 100 mM NaCl, 0.8 mM MgCl₂). After an incubation period of about 20 min optical density (OD) at 405 nm is measured in an MRX microplate reader (Dynatech Laboratories).

All dilutions (except coating antibodies) are prepared in 1% BSA/TBS. All incubations at room temperature are carried out on a rotational ELISA plate shaker (Titramax 100, Heidolph, Bioblock Scientific). All washing steps are performed with TBS/Tween 20 (0.05%, v/v), using an automatic microtiter plate washer (MRW, Dynatech Laboratories).

Recovery rates of recombinant PSP/reg into diluted serum from a healthy volunteer is as follows: 71% at 1:10, 118% at 1:20 and 95% at 1:40 dilution. Intraplate and interplate variance is less than 5% and 10%, respectively for concentrations within the range of the standard (between 0.1 and 3.5 ng/ml).

The test is established with recombinant human PSP/reg1 alpha (SEQ ID NO:1). Recombinant PSP/reg1 beta (SEQ ID NO:2), the second isoform, was made using the same technique. PSP/reg 1 beta is recognized equally well by the ELISA. Therefore, the ELISA is specific for the known PSP/reg family of proteins.

### Test patients for proof of principle

It has been investigated whether PSP/reg can detect infection and is predictive for organ failure and survival peritonitis patients following abdominal surgery.

The study population included 137 patients admitted to the Intensive Care Unit, Universitätsklinikum Magdeburg, with suspicion of peritonitis, in a time period from January 2010 to April 2010. Patients with pancreatic injury were excluded.

Table 1 summarizes demographic data and injury scores at the day of admission. Severity of injury and gender distribution were very similar.

**Table 1: Characteristics of enrolled patients**

| **Patient characteristics** | **All patients n = 137** | **No peritonitis n = 46** | **Peritonitis n = 91** | ***p* value^{†}** |
|---|---|---|---|---|
| Age, median (IQR) | 61 (50-72) | 55 (50-65) | 66 (50-72) | 0.045 |
| Gender, n male/female (%) | 75/61 (55%/45%) | 22/23 (49%/51%) | 53/38 (58%/42%) | 0.198 |
| Peritonitis localisation, n contained/diffuse (%) | 26/65 (19%/52%) | - | 26/65 (29%/71%) | - |
| APACHE II^{a}, score | 15 (11-22) | 11 (8-13) | 18 (14-26) | <0.001 |
| Organ failure, n (%) | 67 (49%) | 6 (13%) | 61 (67%) | <0.001 |
| Non-survivors, n (%) | 23 (17%) | 0 (0%) | 23 (25%) | <0.001 |

| | | | | |
|---|---|---|---|---|
| n: number. ^{a}APACHE II, acute physiology and chronic health evaluation II | | | | |

### Blood status of peritonitis patients - Determination of standard indicators of inflammation

Blood levels of commonly used indicators of inflammation, e.g. IL-6 and procalcitonin (PCT), and C-reactive protein (CRP) were measured at admission to the ICU to demonstrate the extent of inflammation. Although all these parameters were increased in patients with infections, PSP/reg achieved a higher significance level when compared to the other parameters (Table 2).

**Table 2: Standard inflammation indicators at admission.**

| **Patient characteristics** | **All patients n = 137** | **No peritonitis n = 46** | **Peritonitis n = 91** | ***p* value^{†}** |
|---|---|---|---|---|
| White Cell Count (WCC) | 14 (10-19) | 13 (9-17) | 15 (11-20) | 0.079 |
| C-reactive Protein (ng/ml) | 156 (58-249) | 51 (33-86) | 222 (143-291) | <0.001 |
| Interleucin-6 (ng/ml) | 42 (0-174) | 0 (0-0) | 88 (34-375) | <0.001 |
| Procalcitonin (ng/ml) | 0.5 (0.10-2.6) | 0.1 (0.03-0.2) | 1.07 (0.27-6.1) | <0.001 |
| PSP/reg (ng/ml) | 29 (16-204) | 15 (11-23) | 125 (25-419) | <0.0001 |

| | | | | |
|---|---|---|---|---|
| Median (IQR) | | | | |

### PSP/reg is upregulated upon development of peritonitis

It was previously shown that serum PSP/reg levels are increased in patients with severe trauma and no pancreatic injury, and that this increase is significant in polytraumatic patients exhibiting severe sepsis. The present study aims at assessing PSP/reg serum levels of patient with peritonitis.

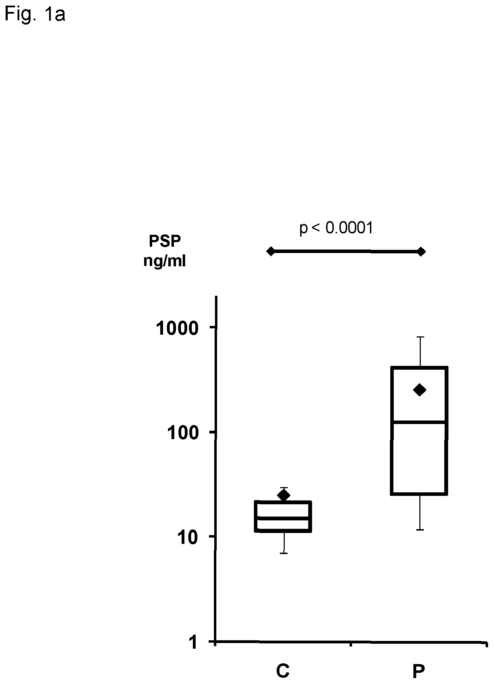

When data are analyzed using stratification assigned to patients without peritonitis and patients with peritonitis, PSP/reg values at admission to the ICU in patients without peritonitis are close to those generally observed in healthy individuals (median: 15 ng/ml), while PSP/reg values are significantly increased in peritonitis patients (median: 125 ng/ml). Thus, PSP/reg discriminates patients who are developing peritonitis from those who are not (Figure 1a).

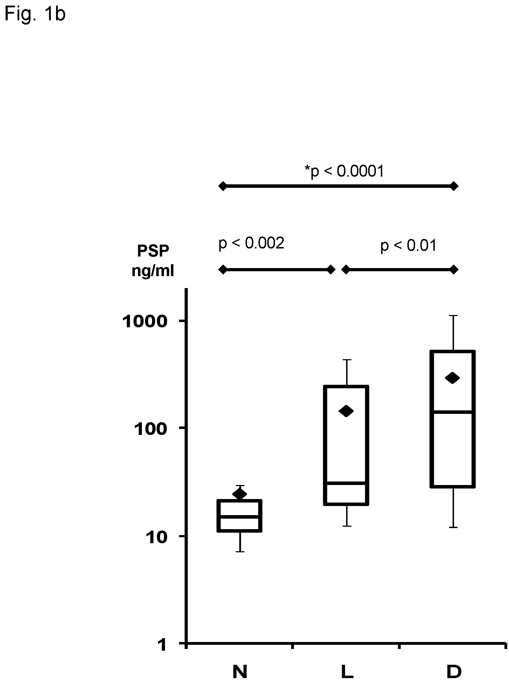

In addition, when data are analyzed using stratification assigned to peritonitis patients with local infection (intra-abdominal abscess) and patients with diffuse infection (intra-abdominal sepsis), PSP/reg values at admission to the ICU in patients with local infection are increased as compared to those observed in patients without infection (median: 31 ng/ml vs. 15 ng/ml, respectively), and PSP/reg values are further increased in patients with diffuse infection (median: 140 ng/ml). Therefore, levels of PSP/reg allow discrimination between local and diffuse peritonitis (Figure 1b).

### Prediction of organ failure

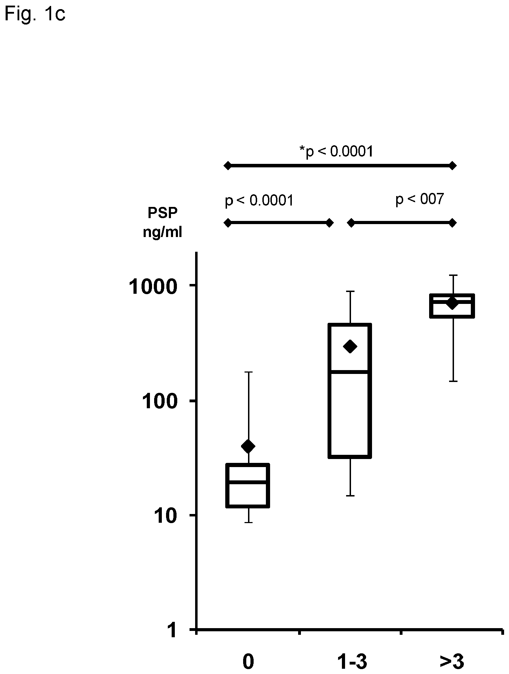

Furthermore, when data are then analyzed using stratification assigned to patients without organ failure, failure of 1 to 3 organs, and failure of more than 3 organs, PSP/reg values at admission to the ICU in patients with no organ failure remain low (median: 19 ng/ml), but are markedly increased in patients with up to three organ failures (median: 183 ng/ml), and are dramatically elevated in patients with failure of more than three organs (median: 825 ng/ml). Therefore, levels of PSP/reg are predictive of the extent of organ disruptions (Figure 1c).

### PSP/reg levels are predictor of outcome

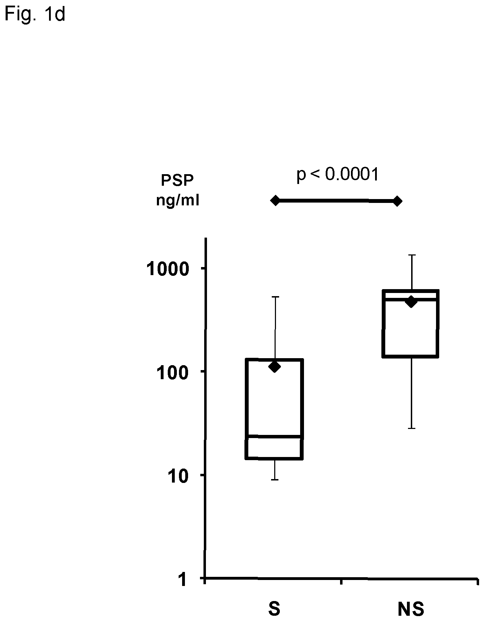

Finally, when data were analyzed using stratification according to survival, PSP/reg values at admission to the ICU were significantly higher in non-survivors (median [IQR]; 499 ng/ml [137-626], n=23) as compared to survivors (24 ng/ml [14-132], n=114; p=0.011) (Figure 1d).

PSP/reg below 25 ng/ml at admission to the ICU was the most accurate threshold for predicting survival. The sensitivity was 53% and specificity 100% for predicting survival (Table 4). In contrast PSP/reg above 400 ng/ml at admission to the ICU was the best cut-off to predict death (sensitivity: 52% specificity: 90%). Positive and negative predictive values were 54% and 89%, accordingly. Patients with PSP/reg above 400 ng/ml at admission to the ICU were at high risk of death within 28 days.

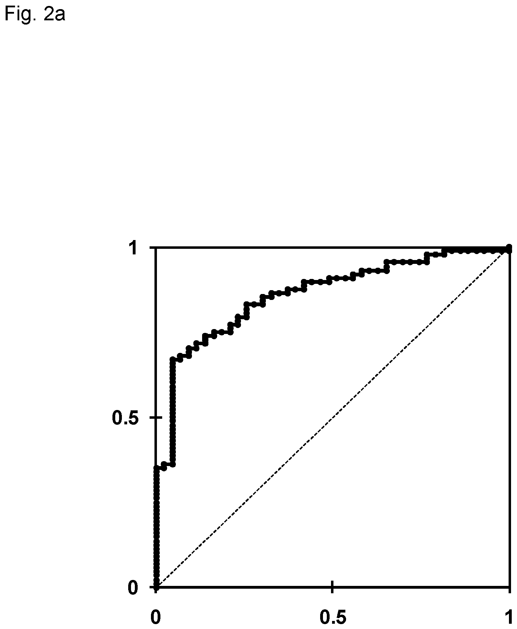

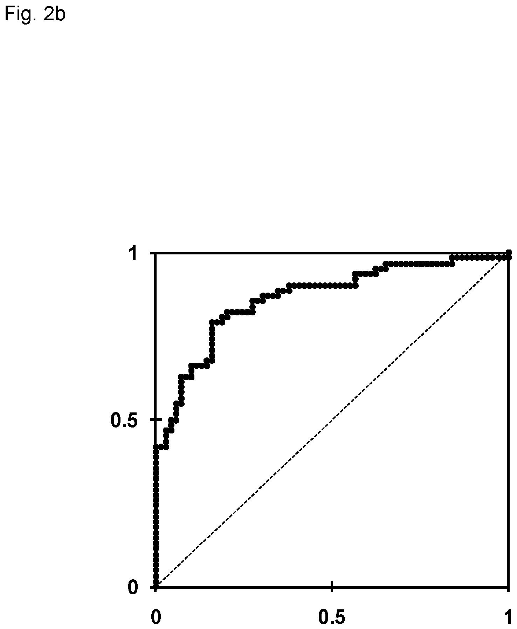

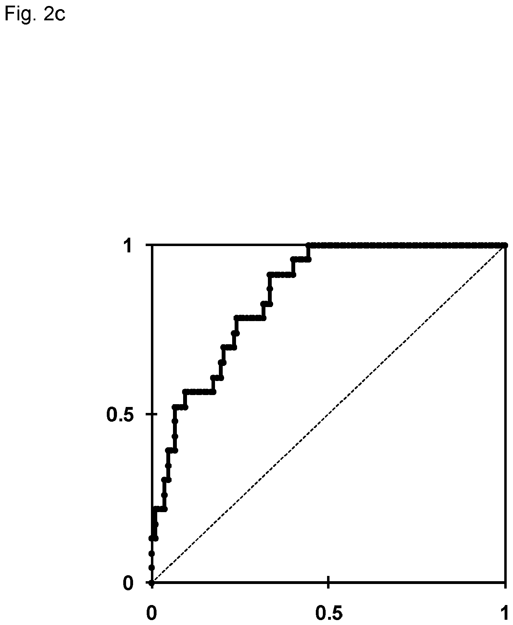

In receiver operating characteristic analysis, the area under the curve of PSP/reg for infectious peritonitis at admission to the ICU is 86% (Figure 2a). For organ failure, the area under the curve of PSP/reg is 87% (Figure 2b). Finally, the area under the curve of PSP/reg for the prediction of mortality/survival at admission to the ICU was 0.847 is 85% (Figure 2c).

In other words, increase of PSP/reg in patients with an intra-abdominal infection can be used as a serum marker to predict the development and the severity of peritonitis and patient outcome. Therefore, specificity and sensitivity are summarized for two potential cut-off values, e.g. 25 and 30 ng/ml, at admission to the ICU. Specificity is above 80 percent for cut-off values of 25 and 30 ng/ml, indicating that patients can be diagnosed and classified by this method shortly after their admission to the ICU.

**Table 3: Sensitivity, specificity and positive and negative predictive values for two cut-off points of PSP/reg serum levels for patients with peritonitis with infection compared with patients without infection.**

| At admission | 25 ng/mL | 30 ng/mL |
|---|---|---|
| Sensitivity | 75% | 67% |
| Specificity | 81% | 95% |
| Positive Predictive Value | 89% | 97% |
| Negative Predictive Value | 61% | 59% |

**Table 4: Sensitivity, specificity and positive and negative predictive values for two cut-off points of PSP serum levels for mortality/survival**

| Day 0 | 25 ng/mL | 30 ng/mL | 400 ng/mL |
|---|---|---|---|
| Sensitivity | 100% | 92% | 52% |
| Specificity | 53% | 64% | 90% |
| Positive Predictive Value | 33% | 37% | 54% |
| Negative Predictive Value | 100% | 97% | 89% |

The analysis is based on PSP/reg serum values obtained at admission to the ICU.

### Severity assessment: PSP/reg correlated with APACHE

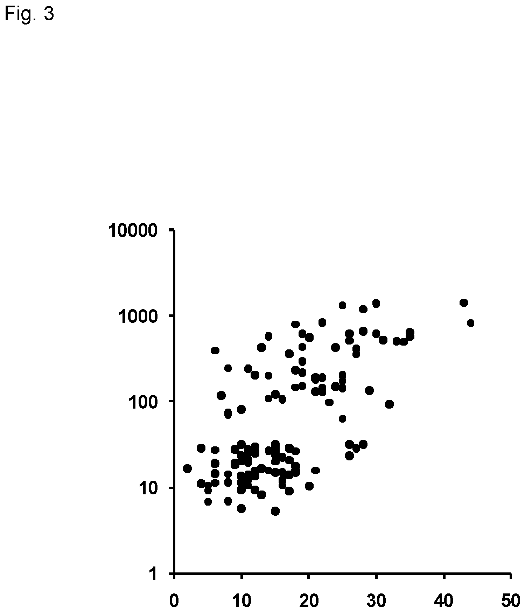

PSP/reg correlated with APACHE II at admission to the ICU (Spearman rank correlation coefficient 0.621; p<0.001, Figure 3).

## Claims

1. An *ex vivo* method of diagnosis of infectious peritonitis in a human, comprising:
a) providing a body fluid sample from said patient, wherein said body fluid sample is a serum sample or a plasma sample;
b) determining the level of pancreatic stone protein / regenerating protein (PSP/reg) in said sample;
c) comparing the level of PSP/reg determined in step b) with a reference value, wherein the reference value is obtained by determining the level of PSP/reg in a body fluid sample from a patient without infection, and wherein the reference value is below 20ng/ml; and
wherein a level of PSP/reg determined in step b) of 30ng/ml or more is indicative of an infectious peritonitis.

2. An *ex vivo* method of predicting the severity and outcome of an infectious peritonitis in a human, comprising:
a) providing a body fluid sample from said patient, wherein said body fluid sample is a serum sample or a plasma sample;
b) determining the level of pancreatic stone protein / regenerating protein (PSP/reg) in said sample;
c) comparing the level of PSP/reg determined in step b) with a reference value, wherein the reference value is obtained by determining the level of PSP/reg in a body fluid sample from a patient without infection, and wherein the reference value is below 20ng/ml;
wherein a level of PSP/reg determined in step b) of 30ng/ml or more is indicative of an infectious peritonitis, and wherein said level of PSP/reg determined in step b) is indicative of the severity of said infection and predictive of the outcome, and wherein a level of PSP/reg equal or higher than 50 ng/ml is indicative of single or multiple organ failure in a patient, and wherein a level of PSP/reg equal or higher than 250 ng/ml is predictive of mortality in a patient.

3. The method of claim 1 or claim 2, wherein said PSP/reg protein has the amino acid sequence of SEQ ID NO: 1 or SEQ ID NO:2.

4. The method of any one of the preceding claims, **wherein said infectious** peritonitis is selected from the group of primary, secondary, or tertiary peritonitis.

5. The method of claim 2, wherein a level of PSP/reg equal or higher than 400 ng/ml is predictive of mortality in a patient.

6. The method of any one of the preceding claims, wherein the level of PSP/reg in said body fluid sample is determined by Enzyme-linked immunosorbent assay (ELISA), Radioimmunoassay (RIA), Enzymoimmunoassay (EIA), mass spectrometry, or microarray analysis.

7. The method of any one of the preceding claims wherein the level of PSP/reg is determined by a sandwich ELISA, wherein microtiter plates are coated with one type of antibody directed against PSP/reg, the plates are then blocked and the sample or standard is loaded, a second type of antibody against PSP/reg is applied, a third antibody detecting the particular type of the second antibody conjugated with a suitable label is then added, and the label used to quantify the amount of PSP/reg.

8. The method of any one of the preceding claims wherein the label in the sandwich ELISA is an enzyme for chromogenic detection.

## Patentansprüche

1. Ex-vivo-Verfahren zur Diagnose von infektiöser Peritonitis bei einem Menschen, umfassend:
a) Bereitstellung einer Körperflüssigkeitsprobe von dem Patienten, wobei es sich bei der Körperflüssigkeitsprobe um eine Serumprobe oder eine Plasmaprobe handelt;
b) Bestimmung des Gehalts an Pankreassteinprotein/regenerierendem Protein (PSP/reg) in der Probe;
c) Vergleichen des Gehalts an in Schritt b) bestimmtem PSP/reg mit einem Referenzwert, wobei der Referenzwert durch Bestimmung des Gehalts an PSP/reg in einer Körperflüssigkeitsprobe von einem Patienten ohne Infektion gewonnen wird und wobei der Referenzwert weniger als 20 ng/ml beträgt und
wobei ein Gehalt von 30 ng/ml oder mehr an in Schritt b) bestimmtem PSP/reg auf eine infektiöse Peritonitis schließen lässt.

2. *Ex-vivo*-Verfahren zur Prognose des Schweregrads und Ausgangs einer infektiösen Peritonitis bei einem Menschen, umfassend:
a) Bereitstellung einer Körperflüssigkeitsprobe von dem Patienten, wobei es sich bei der Körperflüssigkeitsprobe um eine Serumprobe oder eine Plasmaprobe handelt;
b) Bestimmung des Gehalts an Pankreassteinprotein/regenerierendem Protein (PSP/reg) in der Probe;
c) Vergleichen des Gehalts an in Schritt b) bestimmtem PSP/reg mit einem Referenzwert, wobei der Referenzwert durch Bestimmung des Gehalts an PSP/reg in einer Körperflüssigkeitsprobe von einem Patienten ohne Infektion gewonnen wird und wobei der Referenzwert weniger als 20 ng/ml beträgt;
wobei ein Gehalt von 30 ng/ml oder mehr an in Schritt b) bestimmtem PSP/reg auf eine infektiöse Peritonitis schließen lässt und wobei der Gehalt an in Schritt b) bestimmtem PSP/reg auf den Schweregrad der Infektion schließen lässt und eine Prognose auf den Ausgang zulässt und wobei ein Gehalt an PSP/reg von 50 ng/ml oder mehr auf Versagen eines einzelnen oder mehrerer Organe bei einem Patienten schließen lässt und wobei ein Gehalt an PSP/reg von 250 ng/ml oder mehr die Prognose der Mortalität bei einem Patienten bedeutet.

3. Verfahren nach Anspruch 1 oder Anspruch 2, wobei das PSP/reg-Protein die Aminosäuresequenz von SEQ ID NO: 1 oder SEQ ID NO: 2 aufweist.

4. Verfahren nach einem der vorstehenden Ansprüche, wobei die infektiöse Peritonitis aus der Gruppe der primären, sekundären oder tertiären Peritonitis ausgewählt ist.

5. Verfahren nach Anspruch 2, wobei ein Gehalt an PSP/reg von 400 ng/ml oder mehr die Prognose der Mortalität bei einem Patienten bedeutet.

6. Verfahren nach einem der vorstehenden Ansprüche, wobei der Gehalt an PSP/reg in der Körperflüssigkeitsprobe durch enzymgekoppelten Immunosorbens-Assay (ELISA), Radioimmunassay (RIA), Enzymoimmunassay (EIA), Massenspektrometrie oder Mikroarray-Analyse bestimmt wird.

7. Verfahren nach einem der vorstehenden Ansprüche, wobei der Gehalt an PSP/reg durch einen Sandwich-ELISA bestimmt wird, wobei Mikrotiterplatten mit einem gegen PSP/reg gerichteten Antikörpertyp beschichtet werden, die Platten anschließend blockiert werden und die Probe oder der Standard geladen wird, ein zweiter Antikörpertyp gegen PSP/reg angewendet wird, ein dritter Antikörper, der den jeweiligen Typ des mit einem geeigneten Marker konjugierten zweiten Antikörpers ermittelt, anschließend zugegeben wird und der Marker zur Quantifizierung der Menge an PSP/reg verwendet wird.

8. Verfahren nach einem der vorstehenden Ansprüche, wobei es sich bei dem Marker in dem Sandwich-ELISA um ein Enzym für chromogenen Nachweis handelt.

## Revendications

1. Méthode *ex vivo* de diagnostic d'une péritonite infectieuse chez un humain, comprenant :
a) le fait de se munir d'un échantillon de fluide corporel issu dudit patient, où ledit échantillon de fluide corporel est un échantillon de sérum ou un échantillon de plasma ;
b) la détermination du niveau de protéine de régénération de protéine de calcul pancréatique I (PSP/reg) dans ledit échantillon ;
c) la comparaison du niveau de PSP/reg déterminé dans l'étape b) à une valeur de référence, où la valeur de référence est obtenue par détermination du niveau de PSP/reg dans un échantillon de fluide corporel issu d'un patient sans infection, et où la valeur de référence est inférieure à 20 ng/ml ; et
où un niveau de PSP/reg déterminé dans l'étape b) de 30 ng/ml ou plus indique une péritonite infectieuse.

2. Méthode *ex vivo* de prédiction de la sévérité et de l'issue d'une péritonite infectieuse chez un humain, comprenant :
a) le fait de se munir d'un échantillon de fluide corporel issu dudit patient, où ledit échantillon de fluide corporel est un échantillon de sérum ou un échantillon de plasma ;
b) la détermination du niveau de protéine de régénération de protéine de calcul pancréatique I (PSP/reg) dans ledit échantillon ;
c) la comparaison du niveau de PSP/reg déterminé dans l'étape b) à une valeur de référence, où la valeur de référence est obtenue par détermination du niveau de PSP/reg dans un échantillon de fluide corporel issu d'un patient sans infection, et où la valeur de référence est inférieure à 20 ng/ml ;
où un niveau de PSP/reg déterminé dans l'étape b) de 30 ng/ml ou plus indique une péritonite infectieuse, et où ledit niveau de PSP/reg déterminé dans l'étape b) indique la sévérité de ladite infection et en prédit l'issue, et où un niveau de PSP/reg supérieur ou égal à 50 ng/ml indique une insuffisance mono ou multi-organe chez un patient, et où un niveau de PSP/reg supérieur ou égal à 250 ng/ml prédit une issue fatale chez un patient.

3. Méthode selon la revendication 1 ou la revendication 2, où ladite protéine PSP/reg présente la séquence d'acides aminés SEQ ID NO: 1 ou SEQ ID NO:2.

4. Méthode selon l'une quelconque des revendications précédentes, où ladite péritonite infectieuse est choisie dans le groupe des péritonites primaires, secondaires ou tertiaires.

5. Méthode selon la revendication 2, où un niveau de PSP/reg supérieur ou égal à 400 ng/ml prédit une issue fatale au patient.

6. Méthode selon l'une quelconque des revendications précédentes, où le niveau de PSP/reg dans ledit échantillon de fluide corporel est déterminé par dosage immunologique (ELISA), dosage radio-immunologique (RIA), dosage immunologique enzymatique (EIA), spectrométrie de masse ou analyse sur micropuce.

7. Méthode selon l'une quelconque des revendications précédentes, où le niveau de PSP/reg est déterminé par ELISA sandwich, où les plaques de microtitration sont revêtues d'un seul type d'anticorps dirigé contre PSP/reg, les plaques sont ensuite bloquées et l'échantillon ou l'étalon est chargé, un deuxième type d'anticorps contre PSP/reg est appliqué, un troisième anticorps détectant le type spécifique du deuxième anticorps conjugué à un label adapté est ensuite ajouté, et le label est utilisé pour quantifier la quantité de PSP/reg.

8. Méthode selon l'une quelconque des revendications précédentes, où le label de l'ELISA sandwich est une enzyme de détection chromogène.
